# EUROPEAN PATENT APPLICATION

(11) **EP 4 606 339 A1**
(43) Date of publication of application: **27.08.2025**
(21) Application number: 23880213.6
(22) Date of filing: 18.10.2023
(51) Int. Cl.: A61B 34/30, A61B 90/00, A61B 17/00

(54) **CONNECTOR MOUNTER CONNECTED TO MEDICAL INSTRUMENT CONTROL DEVICE**

(30) Priority: 20.10.2022 KR 20220136004; 02.02.2023 KR 20230014245
(71) Applicant: LN Robotics Inc., Seoul 05505 (KR)
(72) Inventor: WON, Jong Seok, Seoul 06226 (KR); KIM, Seong Jun, Seoul 04759 (KR); JEONG, Se Yun, Yongin-si Gyeonggi-do 16914 (KR)
(74) Representative: Lavoix
(86) International application number: PCT/KR2023/016115
(87) International publication number: WO 2024/085636

(57) **Abstract**

In an embodiment, a connector mounter connected to a medical instrument control device comprises: a base plate; a connector holder located at the distal portion of the base plate in order to hold a medical instrument connector; and a medical instrument guide located at the proximal portion of the base plate in order to guide the passage of the medical instrument which passes through the medical instrument connector.

## Description

### TECHNICAL FIELD

The following embodiment relates to a connector mounter connected to a medical instrument control device.

### BACKGROUND ART

In conventional percutaneous coronary intervention (PCI), a practitioner bears the risk of continuous radiation exposure, significant time and costs are required to train a skilled doctor for stable treatment, and it is difficult to commonly provide high-level medical services because of gaps among practitioners, regions, and hospitals in terms of treatment completion level. To compensate for these shortcomings, an intervention procedure assistant robot is introduced. For example, the intervention procedure assistant robot may be configured to move forward, move backward, or rotate a medical instrument when a user inputs a command.

The above description is information the inventor(s) acquired during the course of conceiving the present disclosure, or already possessed at the time, and is not necessarily art publicly known before the present application was filed.

Korean Patent No. 10-2019-0121928 (published on October 29, 2019) discloses a driving device for a medical robot and a medical robot.

### DISCLOSURE OF THE INVENTION

### TECHNICAL GOALS

A goal of an embodiment is to provide a connector mounter to a medical instrument control device to easily and firmly grip a plurality of medical instruments when the medical instrument control device for controlling the plurality of medical instruments for a complex percutaneous coronary intervention (PCI) is provided.

A goal of an embodiment is to provide a connector mounter for anyone to easily perform a task of gripping a medical instrument by a medical instrument control device.

### TECHNICAL SOLUTIONS

In an embodiment, a connector mounter connected to a medical instrument control device may include a base plate, a connector holder positioned at a distal of the base plate to hold a medical instrument connector, and a medical instrument guide positioned at a proximal of the base plate to guide a path of a medical instrument that passes through the medical instrument connector.

In an embodiment, the connector holder may include a holder plate connected to a distal end of the base plate in a hinge manner, and a proximal holder positioned at a proximal of the holder plate to hold the medical instrument connector.

In an embodiment, the connector holder may further include a distal holder positioned on a distal of the holder plate to hold a distal end of the medical instrument connector or hold a medical instrument connected to the distal end of the medical instrument connector.

In an embodiment, the distal holder may be detachably attached to the holder plate.

In an embodiment, the connector holder may further include a support link connected to the holder plate in a hinge manner, and when the holder plate is lifted upward relative to the base plate, an end of the support link may be caught by a support bump formed on the base plate to maintain a lifted state of the holder plate.

In an embodiment, the medical instrument guide may include a lower guide positioned at the proximal of the base plate, and an upper guide connected to the lower guide in a hinge manner.

In an embodiment, the lower guide may include a lower body positioned at the proximal of the base plate, and a valley recessed from the lower body to insert and dispose the medical instrument therein, and the upper guide may include an upper body connected to the lower body in a hinge manner, and a blade protruding from a lower part of the upper body to close an open upper part of the valley.

In an embodiment, the upper guide may further include at least one rib protruding from both sides of the blade to further protrude downward away from the blade, and the lower guide may further include at least one rib insertion groove formed in the lower body to insert the at least one rib therein.

In an embodiment, among the at least one rib, a rib formed on a proximal end of the upper guide may be formed in a shape corresponding to a shape of a proximal end of the lower body.

In an embodiment, the valley may include a plurality of independent valleys formed independently, and a converging valley formed on a distal of the lower body to converge the plurality of independent valleys into one.

In an embodiment, one of the plurality of independent valleys may be formed to be positioned on a same straight line as the converging valley.

In an embodiment, the valley may include a first space of which a width narrows toward a lower side, and a second space of which a width remains the same on a lower side compared to the first space.

In an embodiment, the lower guide may further include a grip guide part formed by recessing at least a portion of a distal end of the lower body.

In an embodiment, the lower guide may further include a protruding wall protruding upward from the lower body at a position where the valley is curved.

In an embodiment, the base plate may include a discharge guide formed on a top surface of the base plate to discharge, in at least one direction, a foreign material that falls onto the base plate from the medical instrument.

In an embodiment, the medical instrument guide may include a plurality of valleys recessed to insert and dispose the medical instrument therein. At least one of the plurality of valleys may be formed in a straight line shape to dispose the medical instrument in a straight line.

Proximal ends of the upper guide and the lower guide may be formed in a shape corresponding to a roller module connected to the medical instrument control device.

At least one of the plurality of independent valleys may include a first section curved in one direction at an angle of 60 degrees to 70 degrees with a radius of curvature of 25 mm to 35 mm from a proximal end of the converging valley, a second section extending in a longitudinal direction by 25 mm to 35 mm from a proximal end of the first section, and a third section curved in the other direction at an angle of 45 degrees to 55 degrees with a radius of curvature of 25 mm to 35 mm from a proximal end of the second section.

At least one of the plurality of independent valleys may include a first section curved in one direction at an angle of 35 degrees to 45 degrees with a radius of curvature of 40 mm to 50 mm from a proximal end of the converging valley, a second section extending in a longitudinal direction by 15 mm to 25 mm from a proximal end of the first section, and a third section curved in the other direction at an angle of 25 degrees to 35 degrees with a radius of curvature of 40 mm to 50 mm from a proximal end of the second section.

### EFFECTS OF THE INVENTION

According to a connector mounter in an embodiment, while preparing a PCI through a medical instrument control device, multiple medical instruments may be easily and securely gripped by the medical instrument control device using the connector mounter.

According to a connector mounter in an embodiment, the mounted medical instrument may be prevented from separating from the medical instrument control device during a procedure.

The effects of the connector mounter according to embodiments are not limited to the above-mentioned effects, and other unmentioned effects can be clearly understood from the following description by one of ordinary skill in the art.

### BRIEF DESCRIPTION OF DRAWINGS

FIG. 1 is a perspective view of a medical instrument control device according to an embodiment.
FIG. 2 is a plan view of a medical instrument control device according to an embodiment.
FIG. 3 is an exemplary usage state view of a medical instrument control device according to an embodiment.
FIG. 4 is a front view of a portion of a roller module of a medical instrument control device according to an embodiment.
FIG. 5 is a perspective view of a connector mounter according to an embodiment.
FIG. 6 is a perspective view illustrating an open state of each component of a connector mounter according to an embodiment.
FIG. 7 is a perspective view of a lower guide according to an embodiment.
FIG. 8 is a plan view of a lower guide according to an embodiment.
FIG. 9 is a bottom perspective view of an upper guide according to an embodiment.
FIG. 10 is a bottom view of an upper guide according to an embodiment.
FIG. 11 is a front view viewing a valley while an upper guide covers a lower guide according to an embodiment.

### BEST MODE FOR CARRYING OUT THE INVENTION

This patent application claims the benefit of patent application No. 10-2022-0136004, filed on October 20, 2022, the entire disclosure of which is incorporated herein by reference for all purposes.

Hereinafter, embodiments will be described in detail with reference to the accompanying drawings. However, various alterations and modifications may be made to the embodiments. Here, the embodiments are not meant to be limited by the descriptions of the present disclosure. The embodiments should be understood to include all changes, equivalents, and replacements within the idea and the technical scope of the disclosure.

The terminology used herein is for the purpose of describing particular example embodiments only and is not to be limiting of the example embodiments. As used herein, the singular forms "a," "an," and "the" are intended to include the plural forms as well, unless the context clearly indicates otherwise. It should be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like constituent elements and a repeated description related thereto will be omitted. In the description of embodiments, detailed description of well-known related structures or functions will be omitted when it is deemed that such description will cause ambiguous interpretation of the present disclosure.

Also, in the description of the components, terms such as first, second, A, B, (a), (b) or the like may be used herein when describing components of the present disclosure. These terms are used only for the purpose of discriminating one constituent element from another constituent element, and the nature, the sequences, or the orders of the constituent elements are not limited by the terms. It should be noted that if one component is described as being "connected," "coupled" or "joined" to another component, the former may be directly "connected," "coupled," and "joined" to the latter or "connected", "coupled", and "joined" to the latter via another component.

The same name may be used to describe an element included in the embodiments described above and an element having a common function. Unless otherwise mentioned, the description of one embodiment may be applicable to other embodiments. Thus, duplicated description is omitted for conciseness.

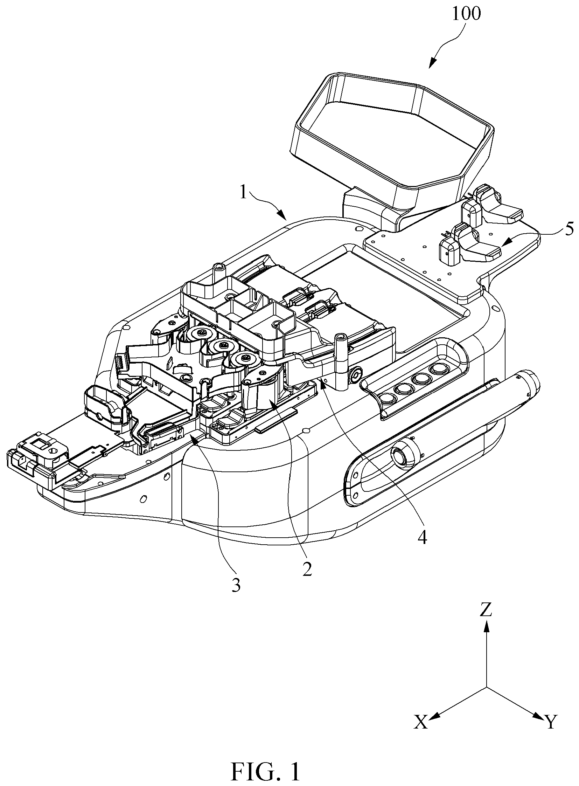

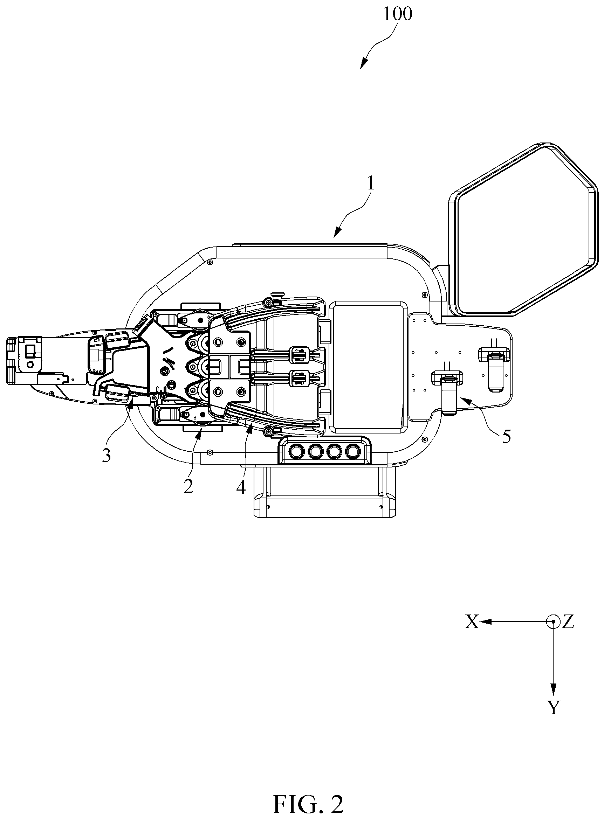

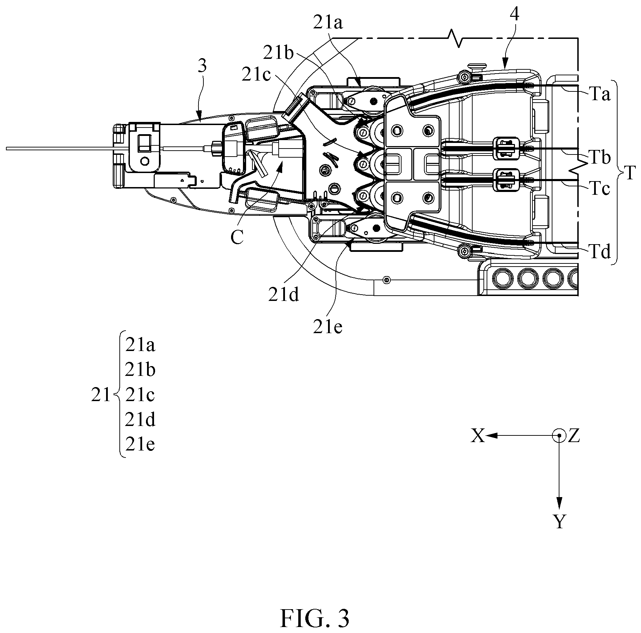

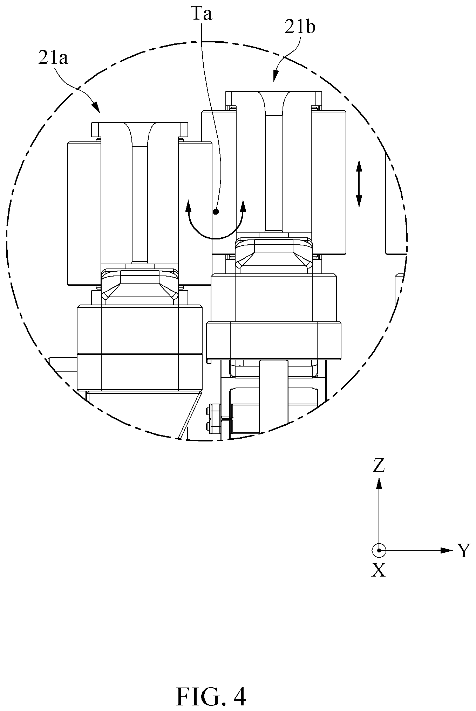

FIG. 1 is a perspective view of a medical instrument control device according to an embodiment. FIG. 2 is a plan view of a medical instrument control device according to an embodiment. FIG. 3 is an exemplary usage state view of a medical instrument control device according to an embodiment. FIG. 4 is a front view of a portion of a roller module of a medical instrument control device according to an embodiment.

Referring to FIGS. 1 to 4, a medical instrument control device 100 may independently control at least one medical instrument T. The medical instrument control device 100 may grip or release the medical instrument T between roller modules 21 described below by horizontal (e.g., the y direction) movement of the roller module 21. The medical instrument control device 100 may implement forward/backward movement and/or rotation of the medical instrument T by rotation and/or vertical (e.g., the z direction) movement of the roller module 21. The medical instrument control device 100 may be used for, for example, percutaneous coronary intervention (PCI). However, this is an example and the purpose of the medical instrument control device 100 is not limited thereto. The medical instrument T may refer to a medical instrument having a longitudinal direction. For example, the medical instrument T may include at least one of a guide wire, a balloon catheter, and a guide catheter having a longitudinal direction. However, this is an example and the type of the medical instrument T is not limited thereto.

Hereinafter, in the description of the medical instrument control device 100, it may be construed that proximal may refer to a side in the -x direction and distal may refer to a side in the +x direction.

The medical instrument control device 100 according to an embodiment may include a housing 1, a driving assembly 2, a connector mounter 3, a medical instrument proximal guide 4, and a rear holder 5.

In an embodiment, the housing 1 may form an exterior of the medical instrument control device 100. The housing 1 may provide an area where the driving assembly 2, the connector mounter 3, the medical instrument proximal guide 4, and/or the rear holder 5 are supported. For example, the housing 1 may be connected to a robotic arm of a slave base. The medical instrument control device 100 may function as an end effector connected to the robotic arm of the slave base. In the housing 1, a handle and/or a switch for adjusting a position of the housing 1 and/or tilting the housing 1 may be provided. In the housing 1, a display panel for conveying information on a state of the device and/or how to use the device to the user may be provided.

In an embodiment, the driving assembly 2 may grip or release the medical instrument T. The driving assembly 2 may move or rotate the gripped medical instrument T in a longitudinal direction.

In an embodiment, the driving assembly 2 may include a plurality of roller modules 21. At least a pair of roller modules 21 may be provided. For example, as shown in the drawing, the roller module 21 may include a first roller module 21a, a second roller module 21b, a third roller module 21c, a fourth roller module 21d, and a fifth roller module 21e. The plurality of roller modules 21 may be arranged parallel to each other. For example, the driving assembly 2 may independently control four medical instruments Ta, Tb, Tc, and Td by five roller modules 21a, 21b, 21c, 21d, and 21e. However, this is an example, and the number of roller modules 21 is not limited thereto.

In an embodiment, the medical instrument T may be gripped between two adjacent roller modules 21. For this, at least one roller module 21 may horizontally move toward the other roller module 21. For example, the second roller module 21b may horizontally move toward the first roller module 21a such that the first medical instrument Ta is gripped between the first roller module 21a and the second roller module 21b. In this case, the second medical instrument Tb positioned between the second roller module 21b and the third roller module 21c may be released. Additionally, on the other hand, the second roller module 21b may horizontally move toward the third roller module 21c such that the second medical instrument Tb is gripped between the second roller module 21b and the third roller module 21c. In this case, the first medical instrument Ta positioned between the first roller module 21a and the second roller module 21b may be released.

Similarly, the fourth roller module 21d may horizontally move toward the third roller module 21c such that the third medical instrument Tc is gripped between the third roller module 21c and the fourth roller module 21d. In this case, the fourth medical instrument Td positioned between the fourth roller module 21d and the fifth roller module 21e may be released. Additionally, on the other hand, the fourth roller module 21d may horizontally move toward the fifth roller module 21e such that the fourth medical instrument Td is gripped between the fourth roller module 21d and the fifth roller module 21e. In this case, the third medical instrument Tc positioned between the third roller module 21c and the fourth roller module 21d may be released.

In an embodiment, the driving assembly 2 may implement forward/backward movement and/or rotation of the medical instrument T by rotation and/or vertical movement of the roller module 21. For example, while the first roller module 21a and the second roller module 21b are positioned adjacent to each other and grip the first medical instrument Ta therebetween, the gripped first medical instrument Ta may move forward or backward in the longitudinal direction by rotating the first roller module 21a and the second roller module 21b in one direction or other directions. In addition, as shown in FIG. 4, while the first roller module 21a and the second roller module 21b are positioned adjacent to each other and grip the first medical instrument Ta therebetween, the gripped first medical instrument Ta may rotate by moving at least one of the roller modules 21a and 21b in the vertical direction.

In an embodiment, the connector mounter 3 may be connected to the housing 1 to be positioned distal (e.g., a side in the +x direction) of the housing 1. The connector mounter 3 may be attached to and detached from the housing 1. The connector mounter 3 may be a consumable and replaceable. The connector mounter 3 may include, for example, a polycarbonate material. However, this is an example, and the material of connector mounter 3 is not limited thereto.

In an embodiment, the connector mounter 3 may hold a medical instrument connector C. For example, the medical instrument connector C may be a component for collecting at least one medical instrument T and guiding a path of the at least one medical instrument T. For example, the medical instrument connector C may include a Y connector. However, this is an example and the type of the medical instrument connector C is not limited thereto. The connector mounter 3 may guide a path of the medical instrument T passing through the medical instrument connector C.

**In** an embodiment, the medical instrument proximal guide 4 may be connected to the housing 1 to be positioned proximal (e.g., a side in the -x direction) compared to the connector mounter 3. The medical instrument proximal guide 4 may be attached to and detached from the housing 1. The medical instrument proximal guide 4 may be a consumable and replaceable. The medical instrument proximal guide 4 may include, for example, a polycarbonate material. However, this is an example and the material of the medical instrument proximal guide 4 is not limited thereto.

In an embodiment, the medical instrument proximal guide 4 may guide a path of the medical instrument T gripped by the driving assembly 2. For example, the medical instrument proximal guide 4 may be connected to the housing 1 to cover at least a portion (e.g., a proximal portion) of the driving assembly 2. The medical instrument proximal guide 4 may include at least one channel to insert and dispose the medical instrument T therein. Each channel may be formed between two adjacent roller modules 21 of the driving assembly 2.

In an embodiment, the rear holder 5 may be connected to the housing 1 to be positioned proximal (e.g., the side in the -x direction) of the housing 1. The rear holder 5 may hold a position of the medical instrument T. For example, the rear holder 5 may include a clamp structure. For example, one rear holder 5 or a plurality of rear holders 5 may be provided. The rear holder 5 may be a consumable and replaceable. The rear holder 5 may include, for example, a polycarbonate material. However, this is an example, and the material of the rear holder 5 is not limited thereto.

In an embodiment, the connector mounter 3, the medical instrument proximal guide 4, and/or the rear holder 5 may be sealed after sterilization and may be provided as disposable products. After the connector mounter 3, the medical instrument proximal guide 4, and/or the rear holder 5 are opened on site for each procedure and are coupled to the housing 1, the connector mounter 3, the medical instrument proximal guide 4, and/or the rear holder 5 may hold the plurality of medical instruments T or may guide the path. When the procedure is terminated, the connector mounter 3, the medical instrument proximal guide 4, and/or the rear holder 5 may be removed from the housing 1 and may be discarded.

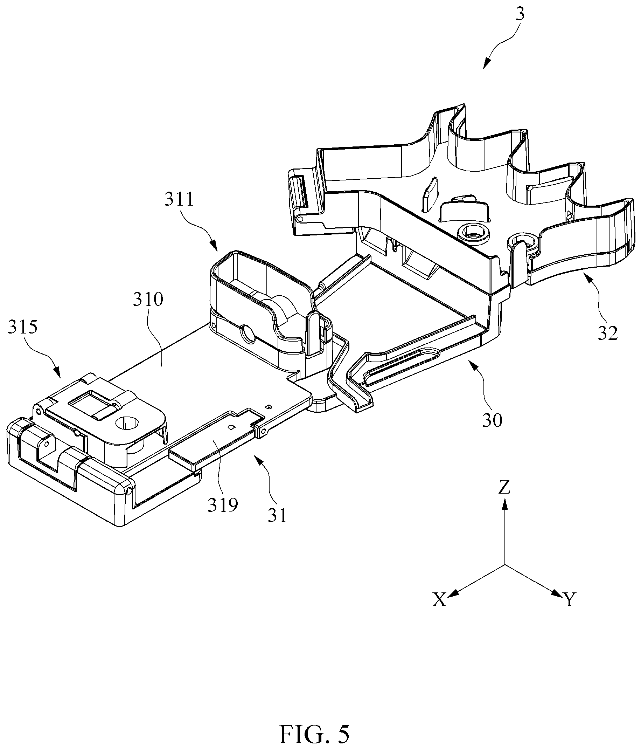

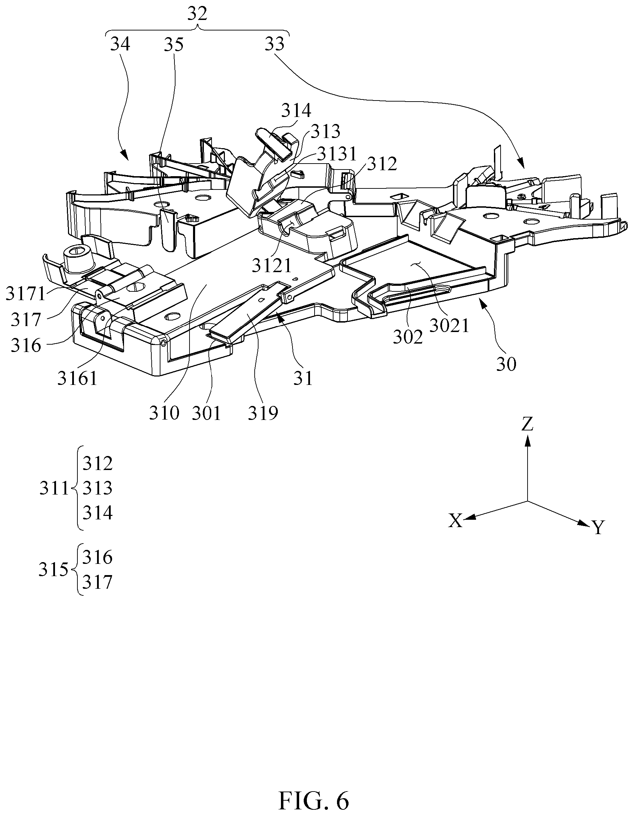

FIG. 5 is a perspective view of a connector mounter according to an embodiment. FIG. 6 is a perspective view illustrating an open state of each component of a connector mounter according to an embodiment.

Hereinafter, the connector mounter 3 connected to the medical instrument control device 100 is described with reference to FIGS. 1 to 6. Hereinafter, in the description of the connector mounter 3, it may be construed that proximal may refer to a side in the - x direction and distal may refer to a side in the +x direction.

The connector mounter 3 in an embodiment may include a base plate 30, a connector holder 31, and a medical instrument guide 32.

In an embodiment, the base plate 30 may form a base of the connector mounter 3. The base plate 30 may be formed as a substantially plate shape. The base plate 30 may be formed long to have a longitudinal direction from the proximal (e.g., the side in the -x direction) to the distal (e.g., the side in the +x direction). However, this is an example and the shape of the base plate 30 is not limited thereto. The base plate 30 may be connected to the housing 1 of the medical instrument control device 100. The base plate 30 may be attached to and detached from the housing 1.

In an embodiment, the connector holder 31 may be a component for holding the medical instrument connector C. The connector holder 31 may be positioned on the distal (e.g., the side in the +x direction) of the base plate 30. For example, the connector holder 31 may be connected in a hinge manner to a distal end (e.g., an end in the +x direction) of the base plate 30.

In an embodiment, the connector holder 31 may include a holder plate 310, a proximal connector holder 311, a distal connector holder 315, and a support link 319.

In an embodiment, the holder plate 310 may form a base of the connector holder 31. The holder plate 310 may be formed as a substantially plate shape. The holder plate 310 may be formed long to have a longitudinal direction from the proximal (e.g., the side in the -x direction) to the distal (e.g., the side in the +x direction). However, this is an example and the shape of the holder plate 310 is not limited thereto.

In an embodiment, the proximal connector holder 311 may be a component for holding the medical instrument connector C. The proximal connector holder 311 may be positioned on the proximal (e.g., the side in the -x direction) of the holder plate 310. For example, the proximal connector holder 311 may be fixedly connected to the holder plate 310. However, this is an example and the proximal connector holder 311 may be attached to and detached from the holder plate 310.

In an embodiment, the proximal connector holder 311 may include a lower proximal connector holder 312, an upper proximal connector holder 313, and a fastening hook 314. The lower proximal connector holder 312 may be connected to the holder plate 310. For example, the lower proximal connector holder 312 may be integrally formed with the holder plate 310. The upper proximal connector holder 313 may be connected to the lower proximal connector holder 312 in a hinge manner. The upper proximal connector holder 313 may rotate between a state in which the upper proximal connector holder 313 covers an upper side (e.g., the side in the +z direction) of the lower proximal connector holder 312 and a state in which the upper proximal connector holder 313 opens the upper side (e.g., the size in the +z direction) of the lower proximal connector holder 312. Grooves 3121 and 3131 for inserting and disposing the medical instrument connector C therein may be formed on the lower proximal connector holder 312 and the upper proximal connector holder 313, respectively. The grooves 3121 and 3131 may be formed in a shape substantially corresponding to the medical instrument connector C. While the medical instrument connector C is disposed on the groove 3121 formed on the lower proximal connector holder 312, the upper proximal connector holder 313 may rotate to cover the lower proximal connector holder 312. While the upper proximal connector holder 313 covers the lower proximal connector holder 312, the upper proximal connector holder 313 and the lower proximal connector holder 312 may be fastened to each other by the fastening hook 314. By the structure described above, the medical instrument connector C may be stably held between the lower proximal connector holder 312 and the upper proximal connector holder 313. However, this is an example and the fastening scheme of the lower proximal connector holder 312 and the upper proximal connector holder 313 is not limited thereto. For example, the lower proximal connector holder 312 and the upper proximal connector holder 313 may be fastened to each other by a magnet.

In an embodiment, the distal connector holder 315 may be a component for holding the medical instrument T (e.g., a guide wire) connected to the medical instrument connector C and/or a distal end (e.g., an end in the +x direction) of the medical instrument connector C. The distal connector holder 315 may be disposed on the distal (e.g., the side in the +x direction) of the holder plate 310. For example, the distal connector holder 315 may be detachably connected to the holder plate 310. For example, the distal connector holder 315 may be attached to and detached from the holder plate 310 by a magnet. By the structure described above, depending on the type of the medical instrument T and/or the medical instrument connector C, an attached position and/or direction of the distal connector holder 315 may be changed, the distal connector holder 315 may be removed, or a different type of distal connector holder 315 may be attached and used, and thereby, the versatility may be improved. However, this is an example and the distal connector holder 315 may be fixedly connected to the holder plate 310.

In an embodiment, the distal connector holder 315 may include a lower distal connector holder 316 and an upper distal connector holder 317. The lower distal connector holder 316 may be connected to the holder plate 310. For example, the lower distal connector holder 316 may be attached to and detached from the holder plate 310 by a magnet. The upper distal connector holder 317 may be connected to the lower distal connector holder 316 in a hinge manner. The upper distal connector holder 317 may rotate between a state in which the upper distal connector holder 313 covers an upper side (e.g., the side in the +z direction) of the lower proximal connector holder 316 and a state in which the upper distal connector holder 313 opens the upper side (e.g., the size in the +z direction) of the lower distal connector holder 316. Grooves 3161 and 3171 for inserting and disposing the medical instrument connector C and/or the medical instrument T therein may be formed on the lower distal connector holder 316 and the upper distal connector holder 317, respectively. The grooves 3161 and 3171 may be formed in a shape substantially corresponding to the medical instrument connector C and/or the medical instrument T. While the medical instrument connector C and/or the medical instrument T are disposed on the groove 3161 formed on the lower distal connector holder 316, the upper distal connector holder 317 may rotate to cover the lower distal connector holder 316. For example, while the upper distal connector holder 317 covers the lower distal connector holder 316, the upper distal connector holder 317 may remain covering the lower distal connector holder 316 as a magnet mounted on the upper distal connector holder 317 is attached to a magnet mounted on the holder plate 310 (or a magnet mounted on the lower distal connector holder 316). By the structure described above, the medical instrument connector C and/or the medical instrument T may be stably held between the lower distal connector holder 316 and the upper distal connector holder 317. However, this is an example and the fastening scheme of the lower distal connector holder 316 and the upper distal connector holder 317 is not limited thereto. For example, the lower distal connector holder 316 and the upper distal connector holder 317 may be fastened to each other by a fastening hook.

In an embodiment, the holder plate 310 may be connected to the distal end (e.g., the end in the +x direction) of the base plate 30 in a hinge manner. For example, the distal end (e.g., the end in the +x direction) of the holder plate 310 and the distal end (e.g., the end in the +x direction) of the base plate 30 may be connected to each other via a hinge. Based on the distal end (e.g., the end in the +x direction) of the base plate 30, a proximal end (e.g., an end in the -x direction) of the holder plate 310 may be lifted upward (e.g., the +z direction) relative to the base plate 30. For example, the holder plate 310 may be lifted by a range of 0 to 6 degrees. However, this is an example and the lifting angle the holder plate 310 is not limited thereto. By the structure described above, the user may lift the connector holder 31 upward (e.g., the side in the +z direction) at a predetermined angle relative to the base plate 30.

In an embodiment, the support link 319 may be connected to the holder plate 310 in a hinge manner. For example, the support link 319 may be connected to the distal (e.g., the side in the +x direction) of the holder plate 310 to be adjacent to the distal connector holder 315. As shown in FIG. 6, when the holder plate 310 is lifted upward (e.g., the side in the +z direction) relative to the base plate 30, an end of the support link 319 may be engaged with a support bump 301 formed on the base plate 30 to maintain the lifted state of the holder plate 310. By the structure described above, when the user lifts the connector holder 31 upward (e.g., the side in the +z direction) relative to the base plate 30, the state may be maintained. In this state, the user may perform a task, such as inserting the medical instrument T into the proximal end (e.g., the end in the -x direction) of the medical instrument connector C. When the task is terminated, the user may return a position of the connector holder 31 to its original position by lifting the support link 319 engaged with the support bump 301 from the support bump 301. By the structure described above, even when the medical instrument connector C is held by the proximal connector holder 311 and/or the distal connector holder 315, the user may perform a task, such as inserting the medical instrument T into the medical instrument connector C by lifting the medical instrument connector C, and thereby, the user convenience may be improved. Meanwhile, the support bump 301 shown in the drawing is an example and a plurality of support bumps 301 may be formed at different positions to fix the holder plate 310 at various angles.

In an embodiment, the base plate 30 may include a discharge guide 302. The discharge guide 302 may be formed on the top surface (e.g., the surface in the +x direction) of the base plate 30 to discharge, in at least one direction, a foreign material (e.g., blood) falling from the medical instrument T onto the base plate 30. For example, the discharge guide 302 may be formed between the proximal connector holder 311 and the medical instrument guide 32. The discharge guide 302 may protrude from the top surface (e.g., the surface in the +x direction) of the base plate 30 to form a dam 3021 of which at least one side is open. A waste disposal bin may be positioned on an open end of the dam 3021. For example, while the medical instrument T inserted into a blood vessel is removed from the blood vessel, the medical instrument T may be stained with a foreign material, such as blood. In this case, the foreign material on the medical instrument T may fall onto the dam 3021 formed on the top surface (e.g., the surface in the +x direction) of the base plate 30. For example, the foreign material, such as blood, may flow out from an inlet of the medical instrument connector C during a procedure and may fall onto the dam 3021 formed on the top surface (e.g., the surface in the +x direction) of the base plate 30. The foreign material that falls onto the dam 3021 may flow in one direction and discharge by the discharge guide 302.

By the structure described above, the medical instrument control device 100 may be prevented from being contaminated by a foreign material (e.g., blood) generated during a procedure.

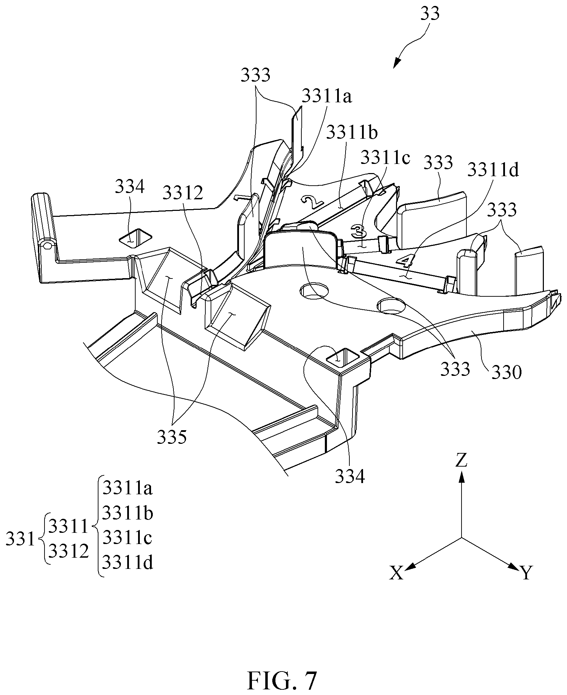

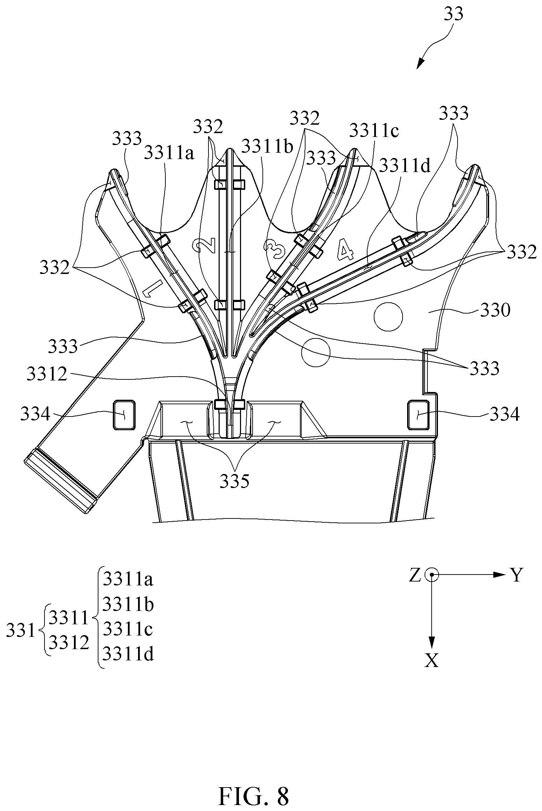

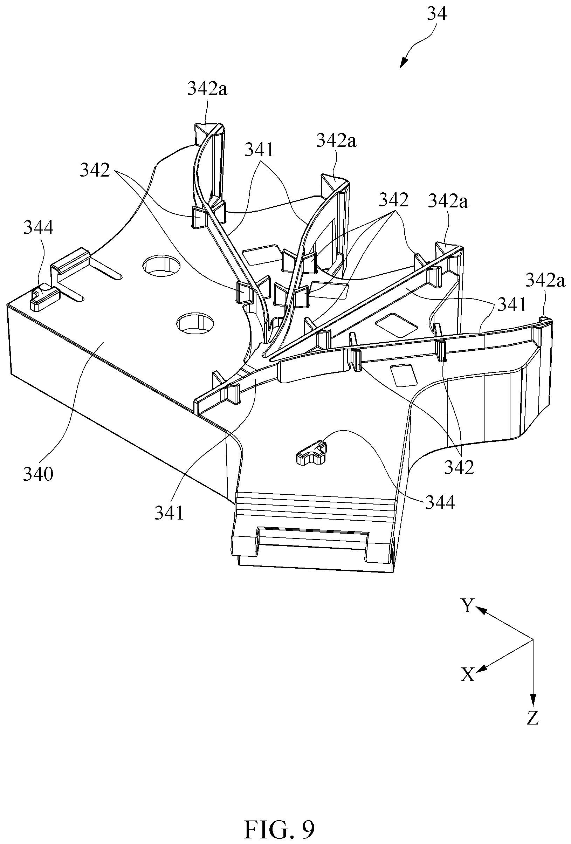

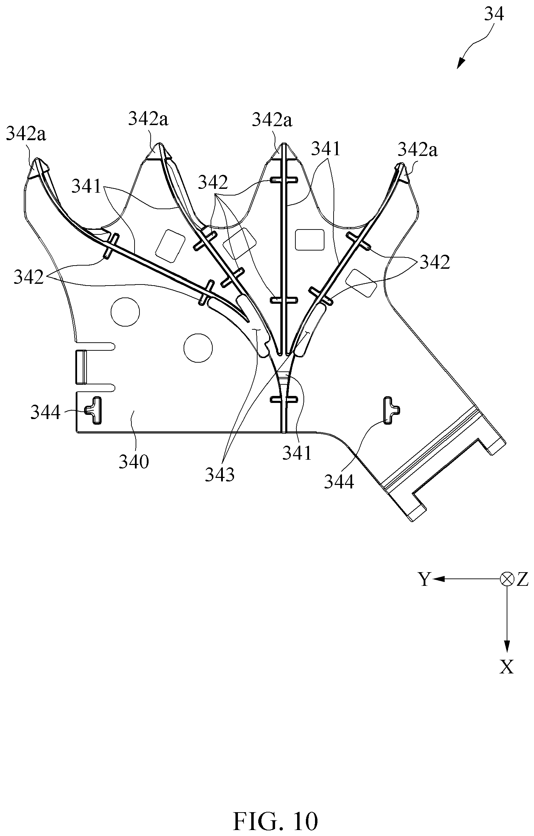

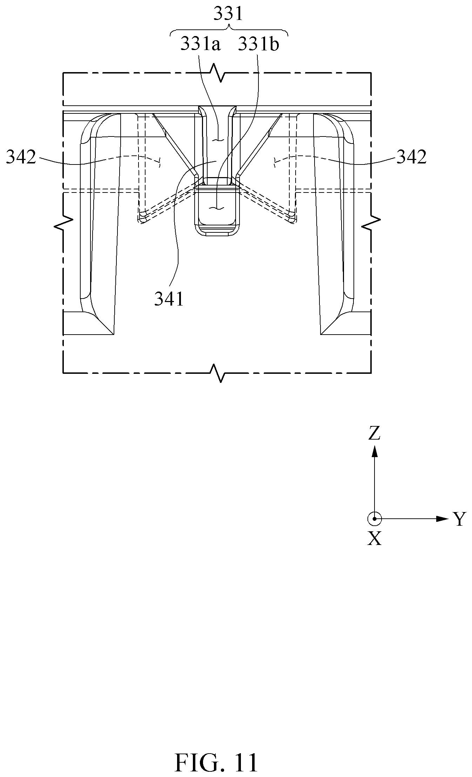

FIG. 7 is a perspective view of a lower guide according to an embodiment. FIG. 8 is a plan view of a lower guide according to an embodiment. FIG. 9 is a bottom perspective view of an upper guide according to an embodiment. FIG. 10 is a bottom view of an upper guide according to an embodiment. FIG. 11 is a front view viewing a valley while an upper guide covers a lower guide according to an embodiment.

Hereinafter, the medical instrument guide 32 according to an embodiment is described with reference to FIGS. 1 to 11. Hereinafter, in the description of the medical instrument guide 32, it may be construed that proximal may refer to a side in the -x direction and distal may refer to a side in the +x direction.

In an embodiment, the medical instrument guide 32 may be a component for guiding a path of the medical instrument T that passes through the medical instrument connector C. The medical instrument guide 32 may be disposed on the proximal (e.g., the side in the -x direction) of the base plate 30.

In an embodiment, the medical instrument guide 32 may include a lower guide 33, an upper guide 34, and a fastening hook 35.

In an embodiment, the lower guide 33 may be positioned on the proximal (e.g., the side in the -x direction) of the base plate 30. For example, the lower guide 33 may be fixedly connected to a proximal end (e.g., the end in the -x direction) of the base plate 30. For example, the lower guide 33 may be integrally formed with the base plate 30.

In an embodiment, the upper guide 34 may be connected to the lower guide 33 in a hinge manner. The upper guide 34 may rotate between a state in which the upper guide 34 covers an upper side (e.g., the side in the +z direction) of the lower guide 33 via a hinge and a state in which the upper guide 34 opens the upper side (e.g., the side in the +z direction) of the lower guide 33.

In an embodiment, the fastening hook 35 may fasten the lower guide 33 and the upper guide 34 to each other. While the upper guide 34 covers the upper side (e.g., the side in the +z direction) of the lower guide 33, the fastening hook 35 may fasten the lower guide 33 and the upper guide 34 to each other. By the fastening hook 35, the state in which the upper guide 34 covers the upper side (e.g., the side in the +z direction) of the lower guide 33 may be maintained. However, this is an example, and the state in which the upper guide 34 covers the upper side (e.g., the side in the +z direction) of the lower guide 33 may be maintained by a magnet.

In an embodiment, the lower guide 33 may include a lower body 330, a valley 331, a rib insertion groove 332, a protruding wall 333, an alignment groove 334, and a grip guide part 335.

In an embodiment, the lower body 330 may form an exterior of the lower guide 33. The lower body 330 may be positioned at the proximal (e.g., the side in the -x direction) of the base plate 30. For example, the lower body 330 may be fixedly connected to the proximal end (e.g., the end in the -x direction) of the base plate 30. For example, the lower body 330 may be integrally formed with the base plate 30. The lower body 330 may be stepped upward (e.g., the +z direction) and connected to the base plate 30 such that the lower body 330 is positioned higher (e.g., the +z direction) than the base plate 30. A proximal end (e.g., the end in the -x direction) of the lower body 330 may be formed in a shape that does not interfere with the driving assembly 2. For example, the proximal end (e.g., the end in the -x direction) of the lower body 330 may be formed in a shape corresponding to the roller module 21 of the driving assembly 2 to insert and dispose the roller module 21 therein.

In an embodiment, the valley 331 may be a space where the medical instrument T is inserted and disposed. The valley 331 may be recessed from a top surface (e.g., the surface in the +z direction) of the lower body 330. The valley 331 may include a plurality of valleys. For example, the valley 331 may include a plurality of independent valleys 3311 and a converging valley 3312. The plurality of independent valleys 3311 may be spaced apart from each other and may be independently formed. The plurality of independent valleys 3311 may extend from the proximal end (e.g., the end in the -x direction) of the lower body 330 toward the distal (e.g., the side in the +x direction). For example, the plurality of independent valleys 3311 may include a first independent valley 3311a, a second independent valley 3311b, a third independent valley 3311c, and a fourth independent valley 3311d. However, this is an example, and the number of independent valleys 3311 is not limited thereto. The converging valley 3312 may be a valley to which the plurality of independent valleys 3311 is converged into one. The converging valley 3312 may be formed on the distal (e.g., the side in the +x direction) of the lower body 330. For example, the converging valley 3312 may be connected to the plurality of independent valleys 3311 and may extend to a distal end (e.g., the end in the +x direction) of the lower body 330.

In an embodiment, one of the plurality of independent valleys 3311 may be positioned on a same straight line as the converging valley 3312. For example, as shown in FIG. 8, the second independent valley 3311b may be positioned on the same straight line as the converging valley 3312. According to the structure described above, the medical instrument T may be arranged in a straight line on at least one of the plurality of valleys 331. However, this is an example and the independent valley 3311 positioned on the same straight line as the converging valley 3312 is not limited thereto. The other independent valleys 3311 (e.g., the first independent valley 3311a, the third independent valley 3311c, and/or the fourth independent valley 3311d) may be curved to converge toward the converging valley 3312. For example, the first independent valley 3311a, the third independent valley 3311c, and/or the fourth independent valley 3311d may be curved once or twice. For example, as the fourth independent valley 3311d extends in a proximal direction (e.g., the -x direction) from the converging valley 3312, the fourth independent valley 3311d may include a first section curved in one direction (e.g., the +y direction) at an angle of 60 degrees to 70 degrees with a radius of curvature of 25 mm to 35 mm from the converging valley 3312, a second section extending in a longitudinal direction by 25 mm to 35 mm from the first section, and a third section curved in the other direction (e.g., the -y direction) at an angle of 45 degrees to 55 degrees with a radius of curvature of 25 mm to 35 mm from the second section. for example, as the third independent valley 3311c extends in the proximal direction (e.g., the -x direction) from the converging valley 3312, the third independent valley 3311c may include a first section curved in one direction (e.g., the +y direction) at an angle of 35 degrees to 45 degrees with a radius of curvature of 40 mm to 50 mm from the converging valley 3312, a second section extending in the longitudinal direction by 15 mm to 25 mm from the first section, and a third section curved in the other direction (e.g., the -y direction) at an angle of 25 degrees to 35 degrees with a radius of curvature of 40 mm to 50 mm from the second section. For example, the first independent valley 3311a may be formed substantially symmetrically with the third independent valley 3311c based on the second independent valley 3311b. According to the shape described above, even when some independent valleys 3311 are curved, when the medical instrument T inserted into the corresponding independent valley 3311 receives a driving force from the driving assembly 2, the medical instrument T may smoothly receive the driving force in the longitudinal direction while without wrinkling in a curved direction. However, this is an example, and the shape of each independent valley 3311 is not limited thereto.

In an embodiment, the valley 331 (e.g., the independent valley 3311 of FIG. 9 and/or the converging valley 3312) may include a first space 331a and a second space 331b. The first space 331a may be a space of which a width narrows toward the lower side (e.g., the -z direction). For example, the first space 331a may have a triangular cross-sectional shape of which the width substantially narrows toward the lower side (e.g., the -z direction). The second space 331b may be positioned lower (e.g., the -z direction) than the first space 331a and may be connected to the first space 331a. The second space 331b may be a space of which the width remains the same. For example, the second space 331b may have a substantially rectangular cross-sectional shape.

In an embodiment, since the width of an upper portion (e.g., a portion in the +z direction) of the first space 331a is relatively wide, the user convenience may be improved when the user inserts the medical instrument T into the valley 331. In addition, since the width of the first space 331a narrows toward the lower side (e.g., the -z direction), the medical instrument T inserted into the upper portion (e.g., the portion in the +z direction) of the first space 331a may be naturally guided and placed on the second space 331b. During the procedure, the medical instrument T may move forward/backward and/or rotate in the second space 331b. Since the second space 331b is formed with the same width, the frequency of the medical instrument T contacting an inner wall of the valley 331 may be reduced while the medical instrument T moves forward/backward and/or rotates in the second space 331b.

In an embodiment, the protruding wall 333 may protrude upward (e.g., the +z direction) from the lower body 330 at a position where the valley 331 is curved. For example, the protruding wall 333 may be formed on an inner portion of a curve at the position where the valley 331 is curved. For example, the protruding wall 333 may be formed along the shape in which the valley 331 is curved. For example, a plurality of protruding walls 333 may be formed. While the user inserts the medical instrument T into the curved valley 331, the protruding wall 333 may guide the medical instrument T to be curved according to the shape of the curved valley 331. For example, since the protruding wall 333 is formed on the curved inner portion of the valley 331, the user may hang the medical instrument T on the protruding wall 333 and may bend the medical instrument T according to the shape of the valley 331. The protruding wall 333 may prevent the medical instrument T inserted into the valley 331 from being ejected from the valley 331 due to its own elasticity at the curved portion. Meanwhile, a wall passing hole 343 may be formed on an upper body 340 of the upper guide 34 to prevent the upper guide 34 from interfering with the protruding wall 333 of the lower guide 33 while the upper guide 34 covers the upper side (e.g., the side in the +z direction) of the lower guide 33. However, this is an example and the wall passing hole 343 may be omitted depending on the height and/or position of the protruding wall 333.

In an embodiment, the grip guide part 335 may be formed by recessing at least a portion of the distal end (e.g., the end in the +x direction) of the lower body 330. The grip guide part 335 may be spaced apart from the converging valley 3312 at both sides (e.g., the sides in the +y and -y directions) of the converging valley 3312. For example, as shown in FIG. 7, the grip guide part 335 may be formed by recessing an upper (e.g., the side in the +z direction) edge of the distal end (e.g., the end in the +x direction) of the lower body 330. The grip guide part 335 may be substantially inclined in a diagonal direction. When the user attempts to lift the medical instrument connector C while the medical instrument connector C is held by the connector holder 31, the grip guide part 335 may reduce interference between a finger of the user and the lower body 330. For example, the user may insert a finger into a space formed by the grip guide part 335 and may grip the proximal end (e.g., the end in the -x direction) of the medical instrument connector C to lift the medical instrument connector C while the medical instrument connector C is held by the connector holder 31. According to the structure described above, the user convenience may be improved.

In an embodiment, the upper guide 34 may include the upper body 340, a blade 341, a rib 342, the wall passing hole 343, and an alignment protrusion 344.

In an embodiment, the upper body 340 may form an exterior of the upper guide 34. The upper body 340 may be connected to the lower body 330 in a hinge manner. The proximal end (e.g., the end in the -x direction) of the upper body 340 may be formed in a shape that does not interfere with the driving assembly 2. For example, the proximal end (e.g., the end in the -x direction) of the upper body 340 may be formed in a shape corresponding to the roller module 21 of the driving assembly 2 to insert and dispose the roller module 21 therein. For example, the upper body 340 may be formed in a shape substantially corresponding to the lower body 330.

In an embodiment, the blade 341 may be a component for closing an open upper part (e.g., the portion in the +z direction) of the valley 331. The blade 341 may protrude from the lower part (e.g., the portion in the -z direction) of the upper body 340. The blade 341 may be formed in a shape substantially corresponding to the valley 331. A length at which the blade 341 protrudes may be less than a depth at which the valley 331 recesses. For example, the length at which the blade 341 protrudes may be greater than the depth of the first space 331a and less than the total depth of the valley 331. For example, a width of the blade 341 may be less than the second space 331b. While the upper guide 34 covers the upper side (e.g., the side in the +z direction) of the lower guide 33, the blade 341 may close the open upper part (e.g., the portion in the +z direction) of the valley 331. According to the structure described above, while the upper guide 34 covers the upper side (e.g., the side in the +z direction) of the lower guide 33, the valley 331 and the blade 341 may form a channel on which the medical instrument T is placed. The medical instrument T may be placed on the channel formed by the valley 331 and the blade 341 and a path thereof may be guided along the channel.

In an embodiment, the rib 342 may protrude from both sides of the blade 341. For example, the rib 342 may be formed in the vertical direction to the longitudinal direction of the blade 341. For example, as shown in FIG. 11, the rib 342 may be formed to further protrude downward (e.g., the -z direction) as the rib 342 moves away from the blade 341. At least one rib 342 may be formed. A plurality of ribs 342 may be positioned apart from each other in the longitudinal direction of the blade 341. While the upper guide 34 covers the upper side (e.g., the side in the +z direction) of the lower guide 33, the rib 342 may be inserted into a rib insertion groove 332 formed on the lower body 330. The position, shape, and/or number of rib insertion grooves 332 may be formed to correspond to the position, shape, and/or number of ribs 342. The rib 342 may prevent the medical instrument T from being caught between the blade 341 and the valley 331 while the medical instrument T is positioned in the valley 331 and the user covers the upper guide 34 with the upper side (e.g., the side in the +z direction) of the lower guide 33. Since the rib 342 further protrudes downward (e.g., the -z direction) as the rib 342 moves away from the blade 341, the rib 342 may guide the medical instrument T to gather the medical instrument T toward the blade 341 while the user covers the upper guide 34 with the upper side (e.g., the side in the +z direction) of the lower guide 33.

In an embodiment, among the at least one rib 342, a proximal rib 342a formed on the proximal end (e.g., the end in the -x direction) of the upper guide 34 may be formed in a shape corresponding to the shape of the proximal end (e.g., the end in the -x direction) of the lower body 330. For example, the proximal rib 342a may be formed in a shape corresponding to an outer part of the proximal end (e.g., the end in the -x direction) of the lower body 330 when the proximal end is viewed from a plane (e.g., the +z direction). The structure described above may prevent the medical instrument T from ejecting from the proximal end (e.g., the end in the -x direction) of the valley 331 and being caught by the blade 341 while the user covers the upper guide 34 with the upper side (e.g., the side in the +z direction) of the lower guide 33.

**In** an embodiment, the alignment protrusion 344 may protrude at the lower part (e.g., the portion in the -z direction) of the upper body 340. While the upper guide 34 covers the upper side (e.g., the side in the +z direction) of the lower guide 33, the alignment protrusion 344 may be inserted into the alignment groove 334 formed on the lower body 330. The position, shape, and/or number of alignment grooves 334 may be formed to correspond to the position, shape, and/or number of alignment protrusions 344. While the user covers the upper guide 34 with the upper side (e.g., the side in the +z direction) of the lower guide 33, the positions of the upper guide 34 and the lower guide 33 may be aligned by inserting the alignment protrusion 344 into the alignment groove 334.

Although the embodiments have been described with reference to the limited drawings, one of ordinary skill in the art may apply various technical modifications and variations based thereon. For example, suitable results may be achieved if the described techniques are performed in a different order and/or if components in a described system, architecture, device, or circuit are combined in a different manner and/or replaced or supplemented by other components or their equivalents.

Accordingly, other implementations are within the scope of the following claims.

## Claims

1. A connector mounter connected to a medical instrument control device, the connector mounter comprising:
a base plate;
a connector holder positioned at a distal of the base plate to hold a medical instrument connector; and
a medical instrument guide positioned at a proximal of the base plate to guide a path of a medical instrument that passes through the medical instrument connector.

2. The connector mounter of claim 1,
wherein the connector holder comprises:
a holder plate connected to a distal end of the base plate in a hinge manner; and
a proximal holder positioned at a proximal of the holder plate to hold the medical instrument connector.

3. The connector mounter of claim 2,
wherein the connector holder further comprises:
a distal holder positioned on a distal of the holder plate to hold a distal end of the medical instrument connector or hold a medical instrument connected to the distal end of the medical instrument connector.

4. The connector mounter of claim 3,
wherein the distal holder is detachably attached to the holder plate.

5. The connector mounter of claim 2,
wherein the connector holder further comprises a support link connected to the holder plate in a hinge manner, and
when the holder plate is lifted upward relative to the base plate, an end of the support link is caught by a support bump formed on the base plate to maintain a lifted state of the holder plate.

6. The connector mounter of claim 1,
wherein the medical instrument guide comprises:
a lower guide positioned at the proximal of the base plate; and
an upper guide connected to the lower guide in a hinge manner.

7. The connector mounter of claim 6,
wherein the lower guide comprises:
a lower body positioned at the proximal of the base plate; and
a valley recessed from the lower body to insert and dispose the medical instrument therein, and
the upper guide comprises:
an upper body connected to the lower body in a hinge manner; and
a blade protruding from a lower part of the upper body to close an open upper part of the valley.

8. The connector mounter of claim 7,
wherein the upper guide further comprises at least one rib protruding from both sides of the blade to further protrude downward away from the blade, and
the lower guide further comprises at least one rib insertion groove formed in the lower body to insert the at least one rib therein.

9. The connector mounter of claim 8,
wherein, among the at least one rib, a rib formed on a proximal end of the upper guide is formed in a shape corresponding to a shape of a proximal end of the lower body.

10. The connector mounter of claim 7,
wherein the valley comprises:
a plurality of independent valleys formed independently; and
a converging valley formed on a distal of the lower body to converge the plurality of independent valleys into one.

11. The connector mounter of claim 10,
wherein one of the plurality of independent valleys is formed to be positioned on a same straight line as the converging valley.

12. The connector mounter of claim 7,
wherein the valley comprises:
a first space of which a width narrows toward a lower side; and
a second space of which a width remains the same on a lower side compared to the first space.

13. The connector mounter of claim 7,
wherein the lower guide further comprises a grip guide part formed by recessing at least a portion of a distal end of the lower body.

14. The connector mounter of claim 7,
wherein the lower guide further comprises a protruding wall protruding upward from the lower body at a position where the valley is curved.

15. The connector mounter of claim 1,
wherein the base plate comprises a discharge guide formed on a top surface of the base plate to discharge, in at least one direction, a foreign material that falls onto the base plate from the medical instrument.

16. The connector mounter of claim 1,
wherein the medical instrument guide comprises a plurality of valleys recessed to insert and dispose the medical instrument therein, and
at least one of the plurality of valleys is formed in a straight line shape to dispose the medical instrument in a straight line.

17. The connector mounter of claim 6,
wherein proximal ends of the upper guide and the lower guide are formed in a shape corresponding to a roller module connected to the medical instrument control device.

18. The connector mounter of claim 10,
wherein at least one of the plurality of independent valleys comprises:
a first section curved in one direction at an angle of 60 degrees to 70 degrees with a radius of curvature of 25 mm to 35 mm from a proximal end of the converging valley;
a second section extending in a longitudinal direction by 25 mm to 35 mm from a proximal end of the first section; and
a third section curved in the other direction at an angle of 45 degrees to 55 degrees with a radius of curvature of 25 mm to 35 mm from a proximal end of the second section.

19. The connector mounter of claim 10,
wherein at least one of the plurality of independent valleys comprises:
a first section curved in one direction at an angle of 35 degrees to 45 degrees with a radius of curvature of 40 mm to 50 mm from a proximal end of the converging valley;
a second section extending in a longitudinal direction by 15 mm to 25 mm from a proximal end of the first section; and
a third section curved in the other direction at an angle of 25 degrees to 35 degrees with a radius of curvature of 40 mm to 50 mm from a proximal end of the second section.
